(19)

Europäisches
Patentamt
European
Patent Office
Office européen
des brevets

(11) **EP 2 433 969 A1**

(12) **EUROPEAN PATENT APPLICATION**
published in accordance with Art. 153(4) EPC

(43) Date of publication:
**28.03.2012 Bulletin 2012/13**

(21) Application number: **10777793.0**

(22) Date of filing: **19.05.2010**

(51) Int Cl.:
***C08B 1/00*** *(2006.01)*      ***A61K 8/73*** *(2006.01)*
***C08B 1/06*** *(2006.01)*

(86) International application number:
**PCT/JP2010/058477**

(87) International publication number:
**WO 2010/134560 (25.11.2010 Gazette 2010/47)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **21.05.2009  JP 2009123074**

(71) Applicant: **Kao Corporation
Chuo-Ku
Tokyo 103-8210 (JP)**

(72) Inventors:
• **OSAKI, Kazutomo
Wakayama-shi
Wakayama 640-8580 (JP)**

• **TOMIOKA, Keiichiro
Wakayama-shi
Wakayama 640-8580 (JP)**
• **NOJIRI, Naoki
Wakayama-shi
Wakayama 640-8580 (JP)**
• **UMEHARA, Masahiro
Wakayama-shi
Wakayama 640-8580 (JP)**
• **MIYAMOTO, Masafumi
Wakayama-shi
Wakayama 640-8580 (JP)**

(74) Representative: **HOFFMANN EITLE
Patent- und Rechtsanwälte
Arabellastraße 4
81925 München (DE)**

(54) **PROCESS FOR PRODUCING NON-CRYSTALLINE CELLULOSE**

(57)      The invention relates to a highly productive process for producing decrystallized cellulose which includes treating a cellulose-containing raw material by means of a mill, wherein the cellulose-containing raw material has a cellulose content of a residue obtained by removing water from the cellulose-containing raw material of 20 mass% or more, has a cellulose I-type crystallinity of cellulose more than 33% as calculated from the following formula (1):

$$\text{Cellulose I-type Crystallinity (\%)} = [(I_{22.6} - I_{18.5})/I_{22.6}] \times 100 \quad (1),$$

wherein $I_{22.6}$ is a diffraction intensity of a lattice plane (002 plane) as measured at a diffraction angle $2\theta$ of 22.6° in X-ray diffraction analysis; and $I_{18.5}$ is a diffraction intensity of an amorphous moiety as measured at a diffraction angle $2\theta$ of 18.5° in X-ray diffraction analysis, and has a water content of 1.8 mass% or less, to thereby reduce the cellulose 1-type crystallinity to 33% or less.

**EP 2 433 969 A1**

**Description**

FIELD OF THE INVENTION

The present invention relates to a process for producing decrystallized celluloses.

BACKGROUND OF THE INVENTION

Celluloses obtained by milling cellulose-containing raw materials such as pulp have been used as industrial materials such as raw materials of cellulose ethers, cosmetics, food stuffs, and biomass materials. Among them, celluloses having a decrystallized crystal structure are particularly useful as these industrial materials.
The crystallinity of cellulose is known to be reduced through, for example, mechanically treating wood material or pulp by means of a mill (refer to, for example, Patent Documents 1 to 7).
Patent Document 1 discloses a method of performing preliminary treatment of wood including adjusting the water content of wood to 3 to 6% and milling the wood by means of a vibration ball mill, to thereby break a microfibril structure of cellulose contained in the wood. Patent Document 2 discloses a multi-step milling method including milling a wood material by means of a vibration ball mill, wherein the wood material is coarsely milled, and the milled wood material is further minutely milled, while the water content of the wood material is adjusted to 2 to 7%.
In Examples 1 and 4 of Patent Document 3, there is disclosed a method of treating sheet-like pulps by means of a vibration ball mill or a twin-screw extruder. In Examples 1 to 3 of Patent Document 4, there is disclosed a method of treating pulp by means of a ball mill. In Examples 1 and 2 of Patent Document 5, there is disclosed a method of treating cellulose powder which has been obtained by subjecting pulp to chemical treatments such as hydrolysis by means of a ball mill and further an air jet mill. However, these methods have failed to achieve satisfactory efficiency and productivity upon reduction of the crystallinity of cellulose.
Patent Documents 6 and 7 disclose a method of producing decrystallized cellulose having a cellulose I-type crystallinity of 33% or less, which method includes treating a cellulose-containing raw material having a bulk density of 100 to 500 $kg/m^3$ by means of a mill filled with balls or rods. However, there is demand for a method which enables further effective reduction in crystallinity of the produced cellulose.
Patent Document 1: JP 62-126999A
Patent Document 2: JP 62-127000A
Patent Document 3: JP 62-236801A
Patent Document 4: JP 2003-64184A
Patent Document 5: JP 2004-331918A
Patent Document 6: Japanese Patent No. 4160108
Patent Document 7: Japanese Patent No. 4160109

SUMMARY OF THE INVENTION

The present invention relates to a process for producing decrystallized cellulose which includes treating a cellulose-containing raw material by means of a mill, wherein the cellulose-containing raw material has a cellulose content of a residue obtained by removing water from the cellulose-containing raw material of 20 mass% or more, has a cellulose 1-type crystallinity of cellulose more than 33% as calculated from the following formula (1):

$$\text{Cellulose I-type Crystallinity (\%)} = [(I_{22.6} - I_{18.5})$$
$$/I_{22.6}] \times 100 \qquad (1),$$

wherein $I_{22.6}$ is a diffraction intensity of a lattice plane (002 plane) as measured at a diffraction angle $2\theta$ of 22.6° in X-ray diffraction analysis; and $I_{18.5}$ is a diffraction intensity of an amorphous moiety as measured at a diffraction angle $2\theta$ of 18.5° in X-ray diffraction analysis, and has a water content of 1.8 mass% or less, to thereby reduce the cellulose I-type crystallinity to 33% or less.

DETAILED DESCRIPTION OF THE INVENTION

The present invention relates to a process for producing decrystallized cellulose having a reduced cellulose I-type crystallinity from a cell ulose-containing raw material in an efficient manner and with an excellent productivity. The inventors have found that the aforementioned problem can be resolved through treatment, by means of a mill, of a cellulose-containing raw material having a water content of 1.8 mass% or less.

Accordingly, the present invention relates to a process for producing decrystallized cellulose which includes treating a cellulose-containing raw material by means of a mill, wherein the cellulose-containing raw material has a cellulose content of a residue obtained by removing water from the cellulose-containing raw material of 20 mass% or more, has a cellulose I-type crystallinity of more than 33% as calculated from the following formula (1):

$$\text{Cellulose I-type Crystallinity (\%)} = [(I_{22.6} - I_{18.5})$$

$$/I_{22.6}] \times 100 \qquad (1),$$

wherein $I_{22.6}$ is a diffraction intensity of a lattice plane (002 plane) as measured at a diffraction angle $2\theta$ of 22.6° in X-ray diffraction analysis; and $I_{18.5}$ is a diffraction intensity of an amorphous moiety as measured at a diffraction angle $2\theta$ of 18.5° in X-ray diffraction analysis, and has a water content of 1.8 mass% or less, to thereby reduce the cellulose I-type crystallinity to 33% or less.

In the present specification, the cellulose I-type crystallinity of cellulose may be referred to simply as a "crystallinity."

[Cellulose-Containing Raw Material]

The cellulose content of a residue obtained by removing water from the cellulose-containing raw material used in the present invention, is 20 mass% or more, preferably 40 mass% or more, more preferably 60 mass% or more. The cellulose content used in the present invention means a total content of cellulose and hemicellulose.

No particular limitation is imposed on the cellulose-containing raw material used in the present invention. Examples of the cellulose-containing raw material include various wood chips; wood materials such as pruned-off branches, thinning wastes, branches, building wastes, and factory wastes; pulp materials such as wood pulp produced from wood materials, and cotton linter pulp obtained from fiber surrounding cotton seeds; paper materials such as newspaper, corrugated cardboard, magazine, and wood-free paper; stems or leaves of plants such as rice straw, and corn stems; and hulls/shells of plants such as chaff, palm shells, and coconut shells. Among them, pulp and wood materials are preferred.

In the commercially available pulp products, the cellulose content of a residue obtained by removing water therefrom is generally from 75 to 99 mass%, and such pulp products contain lignin or the like as another component. Further, the commercially available pulp products usually have a cellulose I-type crystallinity of 60% or more.

[Cellulose I-Type Crystallinity]

The decrystallized cellulose produced according to the present invention has a reduced cellulose 1-type crystallinity of 33% or less. The crystallinity is calculated from diffraction intensity values measured by X-ray diffraction analysis according to the Segal method, and is defined by the following calculation formula (1):

$$\text{Cellulose I-type Crystallinity (\%)} = [(I_{22.6} - I_{18.5})$$

$$/I_{22.6}] \times 100 \qquad (1),$$

wherein $I_{22.6}$ is a diffraction intensity of a lattice plane (002 plane) as measured at a diffraction angle $2\theta$ of 22.6° in X-ray diffraction analysis; and $I_{18.5}$ is a diffraction intensity of an amorphous moiety as measured at a diffraction angle $2\theta$ of 18.5° in X-ray diffraction analysis.

A crystallinity of 33% or less enhances in chemical reactivity of cellulose. For example, when an alkali is added to such a cellulose upon production of cellulose ethers, conversion of the cellulose into an alkali cellulose can readily proceed, resulting in enhanced reaction conversion rate in an etherification reaction of the cellulose. From this viewpoint, the crystallinity, of the decrystallized cellulose is preferably 20% or less, more preferably 15% or less, still more preferably 10% or less, particularly preferably 0%, at which no cellulose I-type crystal is detected upon analysis of the cellulose.

The cellulose I-type crystallinity used herein means a ratio of the I-type crystal of cellulose on the basis of a total amount of a crystalline region of the cellulose. Also, the cellulose I-type means a crystal structure of natural cellulose. The crystallinity of the cellulose has some relation to physical and chemical properties thereof. As crystallinity increases, hardness, density, etc. of cellulose increass, by virtue of a high crystallinity and a less amorphous moiety thereof, but elongation, softness, solubility in water or solvents, and chemical reactivity are lowered.

[Production of decrystallized cellulose]

In the production decrystallized cellulose of the present invention, the aforementioned cellulose-containing raw material having a water content of 1.8 mass% or less is treated with a mill, to thereby reduce the cellulose I-type crystallinity of cellulose to 33% or less (hereinafter the treatment is referred to as "decrystallization treatment"). The water content of the cellulose-containing raw material subjected to the decrystallization treatment of the present invention is 1.8 mass% or less, preferably 1.7 mass% or less, more preferably 1.5 mass% or less, still more preferably 1.2 mass% or less, particularly preferably 1.0 mass% or less. When the water content is 1.8 mass% or less, the cellulose-containing raw material can be readily milled, and the rate of decrystallization by milling is enhanced, whereby the crystallinity can be efficiently reduced in a short period of time. From the viewpoints of productivity and drying efficiency, the lower limit of the water content is preferably 0.2 mass% or more, more preferably 0.3 mass% or more, still more preferably 0.4 mass% or more.

From the above viewpoints, the water content of the cellulose-containing raw material subjected to the decrystallization treatment is preferably 0.2 to 1.8 mass%, more preferably 0.3 to 1.7 mass%, still more preferably 0.4 to 1.5 mass%, particularly preferably 0.4 to 1.0 mass%.

The cellulose-containing raw material subjected to the decrystallization treatment of the present invention preferably has a bulk density of 50 to 600 $kg/m^3$ and a specific surface area of 0.2 to 750 $m^2/kg$.

The bulk density of the cellulose-containing raw material subjected to the decrystallization treatment is preferably 50 $kg/m^3$ or more, more preferably 65 $kg/m^3$ or more, still more preferably 100 $kg/m^3$ or more, in order to more efficiently mill the material for decrystallization. When the bulk density is 50 $kg/m^3$ or more, the cellulose-containing raw material has an appropriate volume, resulting in improved handling property. Further, in such a case, the amount of the raw material fed to the mill can be increased, resulting in enhanced treating capacity of the mill. On the other hand, the upper limit of the bulk density is preferably 600 $kg/m^3$ or less, more preferably 500 $kg/m^3$ or less, still more preferably 400 $kg/m^3$ or less, from the viewpoints of a good handling property and a good productivity. From these viewpoints, the bulk density of the cellulose-containing raw material is preferably 50 to 600 $kg/m^3$, more preferably 65 to 500 $kg/m^3$, still more preferably 100 to 400 $kg/m^3$. Notably, the bulk density may be determined through a method described in the Examples hereinbelow.

The cellulose-containing raw material fed to a mill preferably has a specific surface area of 0.2 to 750 $m^2/kg$ in order to efficiently disperse the raw material into the mill. When the specific surface area is 0.2 $m^2/kg$ or more, the cellulose-containing raw material can be efficiently dispersed in the mill during feeding the raw material into the mill and milled into a desired crystallinity and particle size without requiring a prolonged period of time. On the other hand, the upper limit of the specific surface area is preferably 750 $m^2/kg$ or less, from the viewpoint of productivity. From these viewpoints, the specific surface area is preferably 0.65 to 200 $m^2/kg$, more preferably 0.8 to 50 $m^2/kg$. Notably, the specific surface area may be determined through a method described in the Examples hereinbelow.

Through treating the aforementioned cellulose-containing raw material by means of a mill, the cellulose-containing raw material can be milled so that cellulose can be efficiently decrystallized for a short period of time.

In the case where the cellulose-containing raw material fed to a mill is in the form of chips ($\geq$1 mm $\times$ 1 mm square), the specific surface area is preferably 0.2 to 4 $m^2/kg$, more preferably 0.65 to 3.5 $m^2/kg$, still more preferably 0.8 to 3 $m^2/kg$, in order to efficiently disperse the raw material into the mill.

In the case where the cellulose-containing raw material fed to a mill is in the form of particles (size: $\leq$1 mm), the specific surface area is preferably 3 to 750 $m^2/kg$, more preferably 4.5 to 200 $m^2/kg$, still more preferably 7.5 to 50 $m^2/kg$, in order to enhance productivity and efficiently disperse the raw material into the mill.

[Preliminary treatment performed prior to decrystallization treatment]

In the case where a cellulose-containing raw material having a bulk density less than 50 $kg/m^3$ is used, a preliminary treatment is preferably performed so as to adjust the bulk density to 50 to 600 $kg/m^3$, or the specific surface area to 0.2 to 750 $m^2/kg$. For example, cutting treatment and/or coarse milling may be performed as a preliminary treatment of cellulose-containing raw material, to thereby adjust the bulk density and specific surface area of the cellulose-containing raw material to fall within the above preferred ranges. In order to produce decrystallized cellulose through fewer production steps, cutting treatment is preferably performed as a preliminary treatment of cellulose-containing raw material.

[Cutting treatment]

The cellulose-containing raw material may be cut through an appropriate technique selected in accordance with the type and shape of the cellulose-containing raw material. For example, the cutting means may be one or more cutting machines selected from the group consisting of a shredder, a slitter cutter, and a rotary cutter.

When a sheet-form cellulose-containing raw material is used, a shredder or a slitter cutter is preferably employed as a cutting machine. From the viewpoint of productivity, a slitter cutter is more preferably employed.

When a slitter cutter is employed, a sheet-form raw material is cut along the longitudinal direction thereof by means of a roller cutter, to thereby provide long strips, and the long strips are cut into short pieces along the transverse direction by means of fixed blades and rotary blades, to thereby readily provide dice-form cellulose-containing raw material pieces. As a slitter cutter, a sheet pelletizer available from HORAI Co., Ltd. is preferably employed. By means of this machine, a sheet-form cellulose-containing raw material can be cut into square (about 1 to about 20 mm × about 1 to about 20 mm) pieces.

In the case where a wood material such as thinning waste, pruned-off branch or building waste, or a non-sheet cellulose-containing raw material is cut, a rotary cutter is preferably employed. A rotary cutter includes rotating blades and a screen. By the action of the rotating blades, cut pieces of the cellulose-containing raw material having a size smaller than the opening size of the screen can be readily provided. If required, a fixed blade may be added thereto, and the raw material can be cut by means of the rotating blades and the fixed blades.

When a rotary cutter is employed, the size of the coarsely milled product may be regulated by modifying the opening size of the screen. The opening size of the screen is preferably 1 to 70 mm, more preferably 2 to 50 mm, still more preferably 3 to 40 mm. When the screen has an opening size of 1 mm or more, a coarsely milled product having an appropriate bulk density can be produced, and the handling property thereof is enhanced. When the screen has an opening size of 70 mm or less, the product has a piece size suitable as a raw material to be subjected to a post milling step, and the load of the step can be reduced.

The square piece size of the cellulose-containing raw material after cutting treatment is preferably 1 to 70 mm × 1 to 70 mm, more preferably 2 to 50 mm × 2 to 50 mm. When each piece has a square size of 1 to 70 mm × 1 to 70 mm, a post drying treatment can efficiently and readily performed, and the load required for milling in the post milling treatment can be reduced.

[Coarse milling treatment]

Then, the cellulose-containing raw material, preferably the cellulose-containing raw material pieces obtained through the aforementioned cutting treatment, may be further subjected to a coarse milling treatment in accordance with needs. The coarse milling treatment is preferably performed by means of an extruder, which provides the cellulose material with compression shear force, to thereby break the cellulose crystal structure. Thus, the cellulose-containing raw material is pulverized, whereby the bulk density can be further elevated.

In the method of mechanically milling the cellulose-containing raw material by applying compression shear force thereto, when an impact-type mill which has been generally employed in the conventional technique, for example, a cutter mill, a hammer mill, a pin mill, etc., is employed, the cellulose-containing raw material tends to suffer from flocculation and, therefore, very high bulkiness, resulting in poor handling property and deterioration in mass-based treating capability. On the other hand, when the cellulose-containing raw material is milled by means of an extruder, a milled raw material of interest having a desired bulk density can be produced, resulting in enhanced handling property thereof.

The type of the extruder may be either a single-screw type or a twin-screw type. From the viewpoint of enhancement in conveying capability, etc., among these apparatuses, a twin-screw extruder is preferably employed.

As the twin-screw extruder, there may be used a conventionally known twin-screw extruder in which two screws are rotatably inserted into a cylinder. The rotational directions of the two screws in the twin-screw extruder may be either identical or reverse to each other. From the viewpoint of enhancement in conveying capability, etc., the screws are preferably rotated in the same direction.

The type of meshing of the screws in the extruder may be any of a complete meshing type, a partially meshing type, a de-meshing type. From the viewpoint of enhancement in treating capability, an extruder of a complete meshing type or a partially meshing type is preferred.

From the viewpoint of applying a strong compression shear force to the cellulose-containing raw material, the extruder is preferably provided with a so-called kneading disk segment in any portion of the respective screws thereof. The kneading disk segment consists of a plurality of kneading disks which are continuously arranged in combination while offsetting their positions at a constant phase, for example, at intervals of 90°, and is capable of applying an extremely strong shear force to the cellulose-containing raw material with rotation of the screws by forcibly passing the raw material through a narrow gap between the kneading disks or between the kneading disk and the cylinder. The screw preferably has such a structure that the kneading disk segments and the screw segments are arranged in an alternate relation to each other. In the twin-screw extruder, the two screws are preferably identical in structure to each other.

Upon the coarse milling treatment, preferably, the cellulose-containing raw material, preferably the cellulose-containing raw material pieces obtained through the cutting treatment, is charged into an extruder and continuously treated therein. The shear rate employed upon the treatment is preferably 10 sec$^{-1}$ or more, more preferably 20 to 30,000 sec$^{-1}$, still more preferably 50 to 3,000 sec$^{-1}$, particularly preferably 500 to 3,000 sec$^{-1}$. When the above shear rate is 10 sec$^{-1}$ or more, milling effectively proceeds. The other treating conditions are not particularly limited. The treating temperature is preferably 5 to 200°C.

The number of passes of the cellulose-containing raw material through the extruder may be only one (pass) to attain a sufficient effect. From the viewpoint of reducing the crystallinity and polymerization degree of cellulose, if one pass treatment is unsatisfactory, 2 or more passes are preferably conducted. Also, for attaining high productivity, the number of passes of the cellulose-containing raw material through the extruder is preferably from 1 to 10 (passes). Through repetition of the pass, coarse particles contained in the raw material are milled, thereby obtaining a powdery cellulose-containing raw material having a less fluctuation in particle size. When conducting 2 or more passes, a plurality of the extruders may be arranged in series in consideration of high production capacity.

The average particle size of the cellulose-containing raw material after the coarse milling treatment is preferably 0.01 to 1 mm, in order to efficiently disperse the raw material in the mill for the decrystallization treatment. When the average particle size 1 mm or less, the raw material can be efficiently dispersed in the mill in the decrystallization treatment, whereby the particle size can be adjusted to a desired level without requiring a long period of time. On the other hand, the lower limit of the average particle size is preferably 0.01 mm or more, from the viewpoint of productivity. From these viewpoints, the average particle size is more preferably 0.01 to 0.7 mm, still more preferably 0.05 to 0.5 mm. Notably, the average particle size may be determined through a method described in the Examples hereinbelow.

[Drying treatment]

In the present invention, the cellulose-containing raw material, preferably, the cellulose-containing raw material which has been subjected to the aforementioned cutting treatment and/or coarse milling treatment, is preferably subjected to a drying treatment before the decrystallization treatment.

In general, cellulose-containing raw materials which are generally available as cellulose sources; such as commercially available pulp and biomass resources (e.g., paper, wood, and plant (stems, leaves, husks, etc.)) have a water content in excess of 5 mass%, typically about 5 to about 30 mass%.

Therefore, in the present invention, the water content of the cellulose-containing raw material is preferably adjusted to 1.8 mass% or less through a drying treatment.

The drying method may be appropriately selected from known drying means. Examples of the drying method include the hot-air drying method, the indirect heating drying method, the dehumidifying drying method, the cold-air drying method, the microwave drying method, the IR drying method, the sundrying method, the vacuum drying method, and the freeze drying method.

In any of these drying methods, a known dryer may be appropriately selected and employed therein. For example, a dryer disclosed in "Outline of Particle Technology" (edited by The Association of Powder Process Industry and Engineering, JAPAN, published by The Information Center of Particle Technology, Japan (1995), p. 176) may be employed. These drying methods or drying machines may be employed singly or in combination of two or more members. The drying treatment may be performed batchwise or continuously. However, continuous drying is preferred, from the viewpoint of productivity.

The continuous dryer is preferably a horizontal agitation dryer of an indirect heating type from the viewpoint of thermal conduction efficiency. For preventing micro-dust and attaining stability of continuous discharge, a twin-screw horizontal agitation dryer is more preferred. Examples of the twin-screw horizontal agitation dryer preferably employed in the invention include a twin-screw paddle dryer available from Nara Machinery Co., Ltd.

The temperature at which the drying treatment is performed cannot be unconditionally determined, as it varies depending upon the drying means, drying time, etc. However, the drying temperature is preferably 10 to 250°C, more preferably 25 to 180°C, still more preferably 50 to 150°C. The drying time is preferably 0.01 to 2 hr, more preferably 0.02 to 1 hr. If necessary, drying may be performed under reduced pressure. The pressure is preferably 1 to 120 kPa, more preferably 50 to 105 kPa.

[Decrystallization Treatment]

In the present invention, the media-type mill is preferably employed as a mill for the decrystallization treatment. The media-type mills are classified into a container driving-type mill and a media agitating-type mill.

Examples of the container driving-type mill include a ball mill, a vibration mill, a planetary mill and a centrifugal fluid mill. Among these container driving-type mills, a vibration mill is preferred from the viewpoints of good grinding efficiency and good productivity.

Examples of the media agitating-type mill include tower-type mills such as a tower mill; agitation tank-type mills such as an Attritor, an Aquamizer and a Sand grinder; flow tank-type mills such as a Visco mill and a Pearl mill; flow tube-type mills; annular-type mills such as a co-ball mill; and continuous-type dynamic mills. Among these media agitating-type mills, agitation tank-type mills are preferred from the viewpoints of high grinding efficiency and good productivity. When a media agitating-type mill is employed, the peripheral speed of the tip of agitation blades thereof is preferably from 0.5 to 20 m/s, more preferably from 1 to 15 m/s.

The above types of the mills are described in, for example, "Progress of Chemical Engineering; 30th Collection; Control of Microparticles," Institute of Chemical Engineering, Tokai Division, October 10, 1996, Maki-Shoten.

The treating method may be either a batch method or a continuous method. From the viewpoint of productivity, a continuous method is preferred.

Examples of the media (grinding media) used in the mills include balls, rods and tubes. Among these media, from the viewpoints of high grinding efficiency and good productivity, preferred are balls and rods, with rods being more preferred.

No particular limitation is imposed on the material of the media used in the mills. Examples of the material include iron, stainless steel, alumina, zirconia, silicon carbide, silicon nitride, and glass.

When a vibration mill is employed as a mill with balls serving as a media therefor, the outer diameter of the balls is preferably 0.1 to 100 mm, more preferably 0.5 to 50 mm. When the size of the balls falls within the above-specified range, desired grinding force can be attained, and the cellulose can be efficiently decrystallized without contamination of the cellulose-containing raw material caused by inclusion of fragments of the balls thereinto.

According to the present invention, cellulose contained in the raw material can be efficiently decrystallized through a milling treatment by means of a vibration mill employing rods as a medium, which is advantageous.

Examples of the vibration mill which may be employed in the invention include a Vibro mill available from Uras Techno Co., Ltd., a vibration mill available from Chuo Kakohki Co., Ltd., a small-size vibration rod mill "model 1045" available from Yoshida Seisakusho Co., Ltd., a vibration cup mill "model P-9" available from Fritsch Inc., in Germany, and a small-size vibration mill "model NB-O" available from Nitto Kagaku Co., Ltd.

The rods which may be employed as a medium in the vibration mill are bar-like grinding media, and preferably each have a sectional shape such as a polygonal shape, e.g., a square shape and a hexagonal shape, a circular shape, an elliptical shape, etc.

Preferably, the rods each have an outer diameter of 0.5 to 200 mm, more preferably 1 to 100 mm, still more preferably 5 to 50 mm. No particular limitation is imposed on the length of the rods so long as it is shorter than the length of the container of the mill. When the size of the rods falls within the above-specified range, desired grinding force can be attained, and the cellulose can be efficiently decrystallized without contamination of the cellulose-containing raw material due to inclusion of fragments of the rods thereinto.

The filling ratio of the medium such as balls and rods, which varies depending upon the type of the mill employed, is preferably 10 to 97%, more preferably 15 to 95%. When the filling ratio falls within the above-specified range, the frequency of contact between the cellulose-containing raw material and the medium can be increased, and the grinding efficiency thereof can be enhanced without inhibiting the motion of the grinding media. The "filling ratio" used herein means a ratio of the apparent volume of the medium to the volume of the agitation section of the mill.

The treating time of the mill cannot be unequivocally determined and varies depending upon the type of the mill, the type, size, filling ratio, etc. of the medium such as balls or rods. From the viewpoint of efficiently reducing the crystallinity of the cellulose, the treating time is preferably 0.5 minutes to 24 hours, more preferably 2 minutes to 12 hours, still more preferably 3 minutes to 6 hours, yet more preferably 4 minutes to 1 hour, particularly preferably 5 to 40 minutes.

No particular limitation is imposed on the treating temperature and is preferably 5 to 250°C, more preferably 10 to 200°C, for preventing heat deterioration of cellulose.

Through the aforementioned treating method, decrystallized cellulose having a cellulose 1-type crystallinity of 33% or less can be efficiently produced from the aforementioned cellulose-containing raw material serving as a starting material. In addition, upon the treatment employing the mill, the cellulose-containing raw material can be treated under dry conditions without allowing the milled material to adhere on the inside of the mill.

The average particle size of the resultant decrystallized cellulose is preferably 1 to 150 $\mu$m, more preferably 5 to 100 $\mu$m, still more preferably 7 to 100 $\mu$m, from the viewpoints of good chemical reactivity and good handling property when the decrystallized cellulose is used as an industrial raw material. In particular, decrystallized cellulose having an average particle size of 7 $\mu$m or larger can prevent formation of so-called "undissolved lump or flour" upon contact with a liquid such as water.

[Particle Size Reduction Treatment]

In the present invention, if necessary, the decrystallized cellulose yielded through the decrystallization treatment may further be subjected to a particle size reduction treatment. The particle size reduction treatment may be performed by means of a mill appropriately selected from among known mills, for example, a mill disclosed in "Handbook of Chemical Engineering, revised 6th edition" (edited by The Society of Chemical Engineers, Japan, published by Maruzen Co., Ltd. (1999), p. 843).

These mills may be employed singly or in combination of two or more members. The particle size reduction treatment may be performed in a batch manner or a continuous manner, and a continuous manner is preferred from the viewpoint of productivity.

The mill is preferably a high-speed rotary mill, since it can attain high milling efficiency and a small particle size. Among mills of such a type, a turbo-type mill and an annular mill are more preferred. As a turbo mill, a turbo mill available from Turbo Corporation is preferably employed. As an annular mill, Kryptron Series available from EARTHTECHNICA Co., Ltd. are preferably employed.

In one preferred embodiment of the particle size reduction treatment, the decrystallized cellulose yielded through the decrystallization treatment is fed to a mill and continuously processed by the mill. For yielding decrystallized cellulose having a small particle size, the rotor speed of the high-speed rotary mill is preferably 50 m/s or more, more preferably 100 m/s or more. Although no particular limitation is imposed on the other treatment conditions, the treatment temperature is preferably 5 to 200°C.

[Classification Treatment]

In the present invention, if necessary, the decrystallized cellulose yielded through the decrystallization treatment may further be subjected to a classification treatment. Decrystallized cellulose having a desired particle size can be yielded through classification treatment. The classification treatment may be performed through a technique appropriately selected from known dry classification techniques, such as classification by means of a sieve or pneumatic classification. When the coarse powder obtained through classification, along with the cellulose-containing raw material, is fed to a vibration mill again, and the mixture is subjected to decrystallization treatment, whereby decrystallized cellulose having a small particle size can be efficiently yielded. Examples

The bulk density, specific surface area, average particle size, crystallinity, and water content of the cellulose-containing raw material or decrystallized cellulose as well as the cellulose content thereof were determined through the following methods.

(1) Measurement of Bulk Density

The bulk density was measured using a "Powder Tester" available from Hosokawa Micron Corporation. In the measurement, a sample was fed to a screen being vibrated, and the sample passing the screen was transferred, via a chute, to a standard container (capacity: 100 mL). The weight of the sample in the container was measured, and the bulk density thereof was calculated from the measured value. However, a flocculated sample was fed, via a chute, without passing through a screen, and directly received in a standard container (capacity: 100 mL). Similarly, the weight of the sample in the container was measured, and the bulk density thereof was calculated from the measured value.

(2) Measurement of Specific Surface Area

The specific surface area of the cellulose-containing raw material was determined through the following procedures. In the case where the particles of the raw material had a longer diameter of 1 mm or more, the surface area $A_1$ ($m^2$) and volume $V_1$ ($m^3$) of one particle of the cellulose-containing raw material were determined from an electronic image or by means of a scale, and $A_1/(V_1 \times \rho)$ was calculated (wherein p is a true specific gravity of crystalline cellulose (1,600 kg/$m^3$)). In the case where the cellulose-containing raw material was in the form of chips (square surface area: 1 mm, $\times$ 1 mm or less), the circle-equivalent diameter of one particle of the cellulose-containing raw material was determined from an electronic image, and the surface area $A_1$ ($m^2$) and volume $V_1$ ($m^3$) were calculated from the thus-measured circle-equivalent diameter. Then, $A_1/(V_1 \times \rho)$ was calculated. The thus determined specific surface area values of 100 particles or chips of the cellulose-containing raw material were averaged, to thereby provide the specific surface area of the cellulose-containing raw material.

(3) Measurement of Average Particle Size

The average particle size was measured by means of a laser diffraction/scattering-type particle size distribution measuring device "LA-920" available from Horiba, Ltd. In the measurement, a sample was subjected to an ultrasonic treatment for 1 min prior to measuring the particle size thereof, and the volume-based median diameter of the sample was measured at 25°C by use of water as a dispersing medium.

(4) Calculation of Crystallinity

The cellulose 1-type crystallinity of a sample was calculated from X-ray diffraction intensity values thereof which was measured under the following conditions by means of a "Rigaku PINT 2500VC X-RAY diffractometer" available from Rigaku Corporation, according to the aforementioned calculation formula.

Measuring Conditions:

X-ray source: Cu/Kα-radiation; tube voltage: 40 kV; tube current: 120 mA; measuring range: diffraction angle $2\theta$ = 5 to 45°; X-ray scanning speed: 10°/min; and sample preparation by compressing pellets each having an area of 320 mm$^2$ and a thickness of 1 mm.

(5) Measurement of Water Content

The water content was determined by means of an IR aquameter "MOC-120H" available from Shimadzu Corporation. In the measurement, a sample (5 g) was placed on a weighing tray, and the amount of vaporization was measured at a drying temperature of 120°C in an auto-stop mode (measurement stopped when the variation of water amount in 30 seconds reached 0.05% or less).

(6) Measurement of Cellulose Content

The cellulose content was measured according to a holocellulose determination method as described in "Handbook of Analytical Chemistry," Japan Institute of Analytical Chemistry, revised 4th edition, published on November 30, 1991 from Maruzen Co., Ltd., pp. 1081-1082.

Example 1

[Cutting Treatment]

Wood pulp sheet ["HV-10," available from Tembec Inc., 800 mm × 600 mm × 1.0 mm; crystallinity: 81.5%; cellulose content (a cellulose content of a residue obtained by removing water from the cellulose-containing raw material, hereinafter the same is applied): 96 mass%, and water content: 8.5 mass%] was used as a cellulose-containing raw material. The raw material was cut by means of a sheet pelletizer ("SG(E)-220" available from HORAI Co., Ltd.), to thereby provide chips (about 4 mm × about 4 mm × about 1.0 mm) having a specific surface area of 1.8 m$^2$/kg.

[Drying Treatment]

The pulp material obtained through the cutting treatment was dried by means of a tray dryer (available from ADVANTEC Co.,Ltd., vacuum thermostat drier "DRV320DA") so that the water content of pulp after drying was adjusted to 1.0 mass%. The crystallinity of the pulp material, as calculated from the X-ray diffraction intensity values determined after drying, was 82%. The bulk density of the pulp material measured after drying was 200 kg/m$^3$.

[Decrystallization Treatment]

The pulp material obtained through the drying treatment (100 g) was fed to a batch-type vibration mill ("MB-1" available from Chuo Kakohki Co., Ltd., overall capacity: 3.5 L). Thirteen rods each having a circular cross-section (diameter: 30 mm, length: 218 mm, made of stainless steel) were charged into the mill (filling ratio: 57%) as a milling medium, and the pulp material was processed for 30 minutes at a vibration amplitude of 8 mm and a rotation rate of 1,200 cpm.
After completion of the vibration mill treatment, no pulp-like matter was observed to adhere in the inner wall or bottom of the vibration mill. The thus-yielded decrystallized cellulose was removed from the vibration mill, and the median diameter of the decrystallized cellulose particles was measured. The crystallinity was calculated from the X-ray diffraction intensity values. Table 1 shows the results.

Example 2

The procedure of Example 1 was repeated, except that the pulp material obtained through the cutting treatment was dried to a water content (after drying) of 0.4 mass% in the drying treatment; that a cellulose-containing raw material having a crystlallinity of 79% was used; and that the decrystallization treatment was performed for 15 minutes, to thereby yield decrystallized cellulose. The median diameter of the thus-obtained decrystallized cellulose was measured. The crystallinity was calculated from the X-ray diffraction intensity values. Table 1 shows the results.

Example 3

The procedure of Example 1 was repeated, except that the pulp material obtained through the cutting treatment was dried to a water content (after drying) of 0.6 mass% in the drying treatment, and that a hot-air dryer (QAD, available from Mitsubishi Materials Techno Corporation) was employed as a dryer, to thereby yield decrystallized cellulose. The median diameter of the thus-obtained decrystallized cellulose was measured. The crystallinity was calculated from the X-ray diffraction intensity values. Table 1 shows the results.

Example 4

The procedure of Example 1 was repeated, except that the pulp material obtained through the cutting treatment was dried to a water content (after drying) of 1.7 mass% in the drying treatment, to thereby yield decrystallized cellulose. The median diameter of the thus-obtained decrystallized cellulose was measured. The crystallinity was calculated from the X-ray diffraction intensity values. Table 1 shows the results.

Example 5

[Cutting Treatment]

Rod-shaped, pruned-off branches of Satsuma mandarin trees ($\phi$: 10 mm $\times$ 500 mm, cellulose content: 64 mass%, crystallinity: 46%, and water content: 22 mass%) were milled by means of a plastic mill (Model: JC-2 available from Morita Seiki Kogyo Co., Ltd.), to thereby produce cellulose material chips (about 2 mm $\times$ about 3 mm $\times$ about 1 mm; specific surface area: 2.2 $m^2$/kg) thereof.

[Drying Treatment]

The thus-obtained chips of the cellulose-containing raw material were dried by means of a tray dryer [vacuum thermostat drier "DRV320DA" available from ADVANTEC Co.,Ltd.] so that the water content of the pulp after drying was adjusted to 1.7 mass%.

[Decrystallization Treatment]

The chips of the cellulose-containing raw material obtained through the drying treatment was subjected to a decrystallization treatment. Specifically, the procedure of Example 1 was repeated, except that the number of rods charged into the vibration mill was changed to 11 and that the filling ratio was adjusted to 48%, to thereby yield decrystallized cellulose. The median diameter of the thus-obtained decrystallized cellulose was measured. The crystallinity was calculated from the X-ray diffraction intensity values. Table 1 shows the results.

Comparative Examples 1 and 2

The procedure of Example 1 was repeated, except that the pulp materials obtained through the cutting treatment were dried to a water content (after drying) of 4.9 mass% (Comparative Example 1) and 5.9 mass% (Comparative Example 2) in the drying treatment, respectively, to thereby complete cutting, drying, and decrystallization. The median diameter of the thus-obtained cellulose was measured. The crystallinity was calculated from the X-ray diffraction intensity values. Table 2 shows the results.

Comparative Examples 3 and 4

[Cutting Treatment]

As cellulose-containing raw materials, a mixture of rod-shaped, pruned-off branches of roadside trees (Prunus yedoensis, Quercus phillyraeoides, camphor, Quercus acutissima, and Campsis grandiflora) (Comparative Example 3) ($\phi$: 10 mm $\times$ 300 mm, cellulose content: 67 mass%, crystallinity: 51%, and water content: 12 mass%), and rod-shaped, pruned-off branches of Satsuma mandarin trees (Comparative Example 4) ($\phi$: 10 mm $\times$ 500 mm, cellulose content: 64 mass%, crystallinity: 46%, and water content: 22 mass%) were milled in a manner similar to that employed in Example 5. The thus-obtained chips of each cellulose-containing raw material was subjected to the decrystallization treatment in a manner similar to that employed in Example 5, but was not subjected to a drying treatment, to thereby yield decrystallized cellulose. The median diameter of the thus-obtained cellulose was measured. The crystallinity was calculated from the X-ray diffraction intensity values. Table 2 shows the results.

[Table 1]

| | | Unit | Ex. 1 | Ex. 2 | Ex. 3 | Ex. 4 | Ex. 5 |
|---|---|---|---|---|---|---|---|
| Cellulose-containing raw material | Type of material | | Tembec | Tembec | Tembec | Tembec | Pruned-off branches of mandarin trees |
| | | | Biofloc HV10A | Biofloc HV10A | Biofloc HV10A | Biofloc HV10A | |
| | Form | mm×mm ×mm | 800×600 ×1 | 800×600 ×1 | 800×600 ×1 | 800×600 ×1 | φ10×500 |
| | Cellulose content | mass% | 96 | 96 | 96 | 96 | 64 |
| | Crystallinity | % | 82 | 79 | 82 | 82 | 46 |
| | Water content | mass% | 8.5 | 8.5 | 8.5 | 8.5 | 22 |
| Cutting treatment | Cutter*1 | - | Sheet pelletizer | Sheet pelletizer | Sheet pelletizer | Sheet pelletizer | Plastic mill |
| Raw material after cutting | Form | mm×mm ×mm | 4×4×1 | 4×4×1 | 4×4×1 | 4×4×1 | 2×3×1 |
| Drying treatment | Water content before drying | mass% | 8.5 | 8.5 | 7.9 | 8.5 | 22 |
| | Dryer*2 | - | Tray dryer | Tray dryer | QAD | Tray dryer | Tray dryer |
| Un-decrystallized raw material | Water content | mass% | 1.0 | 0.4 | 0.6 | 1.7 | 1.7 |
| | Crystallinity | % | 82 | 79 | 82 | 82 | 46 |
| | Bulk density | kg/m$^3$ | 200 | 200 | 200 | 200 | 224 |
| | Sp. surface | m$^2$/kg | 1.8 | 1.8 | 1.8 | 1.8 | 2.2 |
| Decrystallization treatment | Mill | - | MB-1 | MB-1 | MB-1 | MB-1 | MB-1 |
| | Medium form | - | Rod | Rod | Rod | Rod | Rod |
| | Medium diam. | mm | 30 | 30 | 30 | 30 | 30 |
| | Medium no. | number | 13 | 13 | 13 | 13 | 11 |

(continued)

| | | Unlit | Ex. 1 | Ex. 2 | Ex. 3 | Ex. 4 | Ex. 5 |
|---|---|---|---|---|---|---|---|
| After (30 min) decrystallization | Median diam. | μm | 51 | 45[*3] | 45 | 59 | 28 |
| | Crystallinity | % | -19 | -30 | -26 | -15 | 0 |

*1: Sheet pelletizer SG(E)-220 available from HORAI Co., Ltd. Plastic mill JC-2 available from Morita Seiki Kogyo Co., Ltd.

*2: QAD (hot-air dryer) available from Mitsubishi Materials Techno Corporation *3: Decrystallization treatment for 15 min

[Table 2]

| | | Unit | Comp. Ex. 1 | Comp. Ex. 2 | Comp. Ex. 3 | Comp. Ex. 4 |
|---|---|---|---|---|---|---|
| Cellulose-containing raw material | Type of material | | Tembec | Tembec | Pruned-off branches of roadside trees | Pruned-off branches of mandarin trees |
| | | | Biofloc HV10A | Biofloc HV10A | | |
| | Form | mm×mm×mm | 800×600×1 | 800×600×1 | φ10×300 | φ10×500 |
| | Cellulose content | mass% | 96 | 96 | 67 | 64 |
| | Crystallinity | % | 82 | 82 | 51 | 46 |
| | Water content | mass% | 8.5 | 8.5 | 12 | 22 |
| Cutting treatment | Cutter[*1] | - | Sheet pelletizer | Sheet pelletizer | Plastic mill | Plastic mill |
| Raw material after cutting | Form | mm×mm×mm | 4×4×1 | 4×4×1 | 2×3×1 | 2×3×1 |
| Drying treatment | Water content before drying | mass% | 8.5 | 8.5 | - | - |
| | Dryer | - | Tray dryer | Tray dryer | - | - |
| Un-decrystallized raw aterial | Water content | mass% | 4.9 | 5.9 | 12 | 22 |
| | Crystallinity | % | 82 | 82 | 51 | 46 |
| | Bulk density | kg/m$^3$ | 200 | 200 | 224 | 224 |
| | Sp. surface area | m$^2$/kg | 1.8 | 1.8 | 2.2 | 2.2 |
| Decrystallization treatment | Mill | - | MB-1 | MB-1 | MB-1 | MB-1 |
| | Medium form | - | Rod | Rod | Rod | Rod |
| | Medium diam. | mm | 30 | 30 | 30 | 30 |
| | Medium no. | number | 13 | 13 | 11 | 11 |

(continued)

| | | Unit | Comp. Ex. 1 | Comp. Ex. 2 | Comp. Ex. 3 | Comp. Ex. 4 |
|---|---|---|---|---|---|---|
| After (30min) decrystallization | Median diam. | μm | 71 | 79 | 109 | 139 |
| | Crystallinity | % | 16 | 18 | 46 | 46 |
| *1: Sheet pelletizer SG(E)-220 available from HORAI Co., Ltd. Plastic mill JC-2 available from Morita Seiki Kogyo Co., Ltd. | | | | | | |

As is clear from Table 1 and 2, the process for producing decrystallized cellulose of Examples 1 to 5 was found to be a highly productive process which is capable of efficiently producing decrystallized cellulose having a reduced cellulose crystallinity for a short period of time, as compared with Comparative Examples 1 to 4.

Comparative Example 5

The procedure of Example 1 was repeated, except that the pulp material obtained through the cutting treatment was dried to a water content (after drying) of 2.0 mass% in the drying treatment, to thereby yield decrystallized cellulose. The median diameter of the thus-obtained decrystallized cellulose was measured. The crystallinity was calculated from the X-ray diffraction intensity values. Table 3 shows the results.

Comparative Example 6

The procedure of Example 1 was repeated, except that the pulp material obtained through the cutting treatment was dried to a water content (after drying) of 3.2 mass% in the drying treatment, to thereby yield decrystallized cellulose. The median diameter of the thus-obtained decrystallized cellulose was measured. The crystallinity was calculated from the X-ray diffraction intensity values. Table 3 shows the results.

Comparative Example 7

The procedure of Example 1 was repeated, except that the pulp material obtained through the cutting treatment was dried to a water content (after drying) of 3.9 mass% in the drying treatment, to thereby yield decrystallized cellulose. The median diameter of the thus-obtained decrystallized cellulose was measured. The crystallinity was calculated from the X-ray diffraction intensity values. Table 3 shows the results.

Comparative Examples 8 and 9

As a cellulose-containing raw material, the same mixture of rod-shaped, pruned-off branches of roadside trees as employed in Comparative Example 3 (Comparative Example 8, water content: 12 mass%), and the same rod-shaped, pruned-off branches of Satsuma mandarin trees as employed in Comparative Example 4 (Comparative Example 9, water content: 22 mass%) were milled in a manner similar to that employed in Example 5.
Subsequently, the thus-obtained chips of pruned-off branches of roadside trees or those of Satsuma mandarin trees were dried by means of a tray dryer [vacuum thermostat drier "DRV320DA" available from ADVANTEC Co.,Ltd.] to a water content thereof to 2.3 mass% and 3.5 mass%, respectively, and each product was subjected to the same decrystallization treatment as carried out in Example 5, to thereby produce decrystallized cellulose. The median diameter of the thus-obtained decrystallized cellulose was measured. The crystallinity was calculated from the X-ray diffraction intensity values. Table 3 shows the results.
[Table 3]

| | | Unit | Comp. Ex. 5 | Comp. Ex. 6 | Comp. Ex. 7 | Comp. Ex. 8 | Comp. Ex. 9 |
|---|---|---|---|---|---|---|---|
| Cellulose-containing raw material | Type of material | | Tembec | Tembec | Tembec | Pruned-off branches of roadside trees | Pruned-off branches of mandarin trees |
| | | | Biofloc HV10A | Biofloc HV10A | Biofloc HV10A | | |
| | Form | mm×mm ×mm | 800×600 ×1 | 800×600 ×1 | 800×600 ×1 | $\phi$10×300 | $\phi$10×500 |
| | Cellulose content | mass% | 96 | 96 | 96 | 67 | 64 |
| | Crystallinity | % | 82 | 82 | 82 | 51 | 46 |
| | Water content | mass% | 8.5 | 8.5 | 8.5 | 12 | 22 |
| Cutting treatment | Cutter*1 | - | Sheet pelletlzer | Sheet pelletizer | Sheet pelletizer | Plastic mill | Plastic mill |
| Raw material after cutting | Form | mm×mm ×mm | 4×4×1 | 4×4×1 | 4×4×1 | 2×3×1 | 2×3×1 |
| Drying treatment | Water content before drying | mass% | 8.5 | 8.5 | 8.5 | 12 | 22 |
| | Dryer | - | Tray dryer | Tray dryer | Tray dryer | Tray dryer | Tray dryer |
| Un-decrystallized raw matenal | Water content | mass% | 2.0 | 3.2 | 3.9 | 2.3 | 3.5 |
| | Crystallinity | % | 82 | 82 | 82 | 51 | 46 |
| | Bulk density | kg/m$^3$ | 200 | 200 | 200 | 224 | 224 |
| | surface area | m$^2$/kg | 1.8 | 1.8 | 1.8 | 2.2 | 2.2 |
| Decrystallization treatment | Mill | - | MB-1 | MB-1 | MB-1 | MB-1 | MB-1 |
| | Medium form | - | Rod | Rod | Rod | Rod | Rod |
| | Medium diam. | mm | 30 | 30 | 30 | 30 | 30 |
| | Medium no. | number | 13 | 13 | 13 | 11 | 11 |
| After (30 min) decrystallization | Median diam. | $\mu$m | 64 | 67 | 74 | 34 | 26 |
| | Crystallinity | % | -13 | -2 | 7.5 | 2 | 8 |

*1: Sheet pelletizer SG(E)-220 available from HORAI Co., Ltd. Plastic mill JC-2 available from Morita Seiki Kogyo Co., Ltd.

As is clear from Table 3, the decrystallized cellulose products obtained by Comparative Examples 5 to 9 each have a relatively large median diameter and high crystallinity, due to high water content of the corresponding raw material before the decrystallization treatment. Thus, in Example 1, the median diameter and crystallinity were found to be reduced to a favorable extent, as compared with Comparative Examples 5 to 9.

Referential Example 1

[Drying Treatment by Means of a Twin-Screw Horizontal Agitation Dryer]

The pulp material obtained through the cutting treatment performed in Example 1 was continuously fed at 21 kg/h to a twin-screw paddle dryer ("NPD-1.6W-1/2L" available from Nara Machinery Co., Ltd., capacity: 47 L, heat transfer area: 1.445 $m^2$) which was heated by steam (0.18 MPa, 130°C) as a heating medium. The pulp material was dried for 47 minutes by the dryer. The dried product was constantly discharged through the discharge outlet at 21 kg/h. The dried pulp product was found to have a water content of 0.7 mass%. The micropowder recovered after drying from the inner wall of the drier and the bag filter was found to be 0.6 mass% with respect to the amount of the raw material fed to the drier.

Referential Example 2

[Drying Treatment by Means of a Single-Screw Disk Dryer]

The pulp material obtained through the cutting treatment performed in Example 1 was continuously fed at 21 kg/h to a single-screw disk dryer ("FDK-60LDK" available from Mitsubishi Materials Techno Corporation, capacity: 60 L, heat transfer area: 1.4 $m^2$) which was heated by steam (0.18 MPa, 130°C) as a heating medium. The pulp material was dried for 90 minutes by the dryer. The discharge flow of the dried product gradually increased from start of drying and reached 21 kg/h 60 minutes after the start. The dried pulp product was found to have a water content of 1.1 mass%. The micropowder recovered after drying from the inner wall of the drier and the bag filter was found to be 1.1 mass% with respect to the amount of the raw material fed to the drier.

Referential Example 3

[Decrystallization Treatment]

The procedure of Example 1 was repeated, except that the pulp material obtained through the cutting treatment was dried to a water content (after drying) of 0.8 mass% in the drying treatment, and that the decrystallization treatment was performed for 13 minutes, to thereby yield decrystallized cellulose. The thus-obtained decrystallized cellulose was found to have a median diameter of 59 $\mu$m and a crystallinity of 15%.

[Particle Size Reduction Treatment by Means of Annular Mill]

The decrystallized cellulose having a median diameter of 59 $\mu$m and produced through the decrystallization treatment was continuously fed at 18 kg/h to a Kryptron (model "KTM-0" available from EARTHTECHNICA Co., Ltd.) with a rotor speed of 135 m/s, to thereby perform the particle size reduction treatment. The resultant cellulose material was found to have a median diameter of 25 $\mu$m.

Referential Example 4

[Particle Size Reduction Treatment by Means of Turbo Mill]

The decrystallized cellulose having a median diameter of 64 $\mu$m and produced through the decrystallization treatment in Referential Example 3 was continuously fed at 60 kg/h to a turbo mill (model "T400-RS" available from Turbo Corporation) with a rotor speed of 157 m/s, to thereby perform the particle size reduction treatment. The cellulose material produced through the particle size reduction treatment was found to have a median diameter of 23 $\mu$m.

As indicated by Referential Examples 1 and 2, Referential Example 1, in which a twin-screw horizontal agitation dryer was employed, was found to more effectively reduce the amount of micropowder and water content. Also, as indicated by Referential Examples 3 and 4, the particle size of the decrystallized cellulose produced through the process of the present invention can be more reduced by means of a mill. The thus-particle-size-reduced decrystallized cellulose is preferably employed as, for example, a reinforce material for resins.

[INDUSTRIAL APPLICABILITY]

The process of the present invention for producing decrystallized cellulose attains excellent productivity and can efficiently produce decrystallized cellulose having a cellulose 1-type crystallinity which is reduced to 33% or less. Thus, the process of the invention is of great value as an industrial production process. The decrystallized cellulose material produced through this process is a particularly useful industrial material such as raw material for cellulose ether, cosmetics, food stuffs, biomass materials, or resin reinforcements.

**Claims**

1.  A process for producing decrystallized cellulose which comprises treating a cellulose-containing raw material by means of a mill, wherein the cellulose-containing raw material has a cellulose content of a residue obtained by removing water from the cellulose-containing raw material of 20 mass% or more, has a cellulose 1-type crystallinity of cellulose more than 33% as calculated from the following formula (1):

$$\text{Cellulose I-type Crystallinity (\%)} = [(I_{22.6} - I_{18.5}) / I_{22.6}] \times 100 \qquad (1),$$

    wherein $I_{22.6}$ is a diffraction intensity of a lattice plane (002 plane) as measured at a diffraction angle 2θ of 22.6° in X-ray diffraction analysis; and $I_{18.5}$ is a diffraction intensity of an amorphous moiety as measured at a diffraction angle 2θ of 18.5° in X-ray diffraction analysis, and has a water content of 1.8 mass% or less, to thereby reduce the cellulose I-type crystallinity to 33% or less.

2.  The process for producing decrystallized cellulose according to claim 1, wherein the cellulose-containing raw material has a bulk density of 50 to 600 kg/m$^3$.

3.  The process according to claim 1 or 2, wherein the cellulose-containing raw material has a specific surface area of 0.2 to 750 m$^2$/kg.

4.  The process for producing decrystallized cellulose according to any of claims 1 to 3, wherein the cellulose-containing raw material is treated by means of a mill for 0.5 minutes to 24 hours.

5.  The process for producing decrystallized cellulose according to any of claims 1 to 4, wherein the mill is a media-agitating mill.

6.  The process for producing decrystallized cellulose according to any of claims 1 to 5, wherein the cellulose-containing raw material is produced through a cutting treatment by means of at least one member selected from the group consisting of a shredder, a slitter cutter and a rotary cutter.

7.  The process for producing decrystallized cellulose according to any of claims 1 to 6, wherein the water content of the cellulose-containing raw material is reduced to 1.8 mass% or less through a drying treatment.

8.  The process for producing decrystallized cellulose according to any of claims 1 to 7, wherein the cellulose-containing raw material is at least one member selected from the group consisting of pulp, paper, stems and leaves of plants, hulls/shells of plants and wood.

9.  Use of decrystallized cellulose produced through a process as recited in any of claims 1 to 8 for a raw material for producing cellulose ether.

10. Use of decrystallized cellulose produced through a process as recited in any of claims 1 to 8 for a cosmetic product.

## INTERNATIONAL SEARCH REPORT

| International application No. |
| --- |
| PCT/JP2010/058477 |

A. CLASSIFICATION OF SUBJECT MATTER
*C08B1/00*(2006.01)i, *A61K8/73*(2006.01)i, *C08B1/06*(2006.01)i

According to International Patent Classification (IPC) or to both national classification and IPC

B. FIELDS SEARCHED

Minimum documentation searched (classification system followed by classification symbols)
C08B1/00, A61K8/73, C08B1/06

Documentation searched other than minimum documentation to the extent that such documents are included in the fields searched
Jitsuyo Shinan Koho        1922-1996   Jitsuyo Shinan Toroku Koho   1996-2010
Kokai Jitsuyo Shinan Koho  1971-2010   Toroku Jitsuyo Shinan Koho   1994-2010

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | WO 2008/099929 A1  (Kao Corp.), 21 August 2008 (21.08.2008), entire text (particularly, paragraph [0007]) & JP 2009-161718 A      & EP 2112169 A1 | 1-10 |
| Y | Cellulose no Kagaku, 2nd print, Asakura Publishing Co., Ltd., 20 April 2005 (20.04.2005), pages 96 to 97 | 1-10 |
| Y | JP 57-011290 A  (Kohjin Co., Ltd.), 20 January 1982 (20.01.1982), claim 1; examples 1 to 3 (Family: none) | 1-10 |

| ☒ Further documents are listed in the continuation of Box C. | ☐ See patent family annex. |
| --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 08 June, 2010 (08.06.10) | 22 June, 2010 (22.06.10) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

| International application No. |
|---|
| PCT/JP2010/058477 |

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 62-127000 A  (Director General, Agency of Industrial Science and Technology, Kobe Steel, Ltd.), 09 June 1987 (09.06.1987), page 5, upper left column (Family: none) | 1-10 |
| P,X | JP 2010-047622 A  (Kao Corp.), 04 March 2010 (04.03.2010), entire text (particularly, claims 1 to 3; examples 1 to 3) (Family: none) | 1-10 |
| A | JP 4160108 B1  (Kao Corp.), 01 October 2008 (01.10.2008), entire text & JP 2009-161717 A | 1-10 |
| A | JP 4160109 B1  (Kao Corp.), 01 October 2008 (01.10.2008), entire text & JP 2009-161718 A     & EP 2112169 A1 & WO 2008/099929 A1 | 1-10 |
| P,A | JP 2010-037526 A  (Kao Corp.), 18 February 2010 (18.02.2010), entire text (Family: none) | 1-10 |

Form PCT/ISA/210 (continuation of second sheet) (July 2009)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62126999 A
- JP 62127000 A
- JP 62236801 A
- JP 2003064184 A

- JP 2004331918 A
- JP 4160108 B
- JP 4160109 B

**Non-patent literature cited in the description**

- Outline of Particle Technology. The Information Center of Particle Technology, 1995, 176
- Progress of Chemical Engineering; 30th Collection; Control of Microparticles. Maki-Shoten, 10 October 1996

- Handbook of Chemical Engineering. Maruzen Co., Ltd, 1999, 843
- Handbook of Analytical Chemistry. Maruzen Co., Ltd, 30 November 1991, 1081-1082